# EUROPÄISCHE PATENTSCHRIFT

(11) **EP 0 909 168 B1**
(45) Veröffentlichungstag und Bekanntmachung des Hinweises auf die Patenterteilung: **21.04.2004**
(21) Anmeldenummer: 97922913.5
(22) Anmeldetag: 25.04.1997
(51) Int. Cl.: A61K 9/72

(54) **TREIBMITTELMISCHUNGEN UND AEROSOLE FÜR DIE MIKRONISIERUNG VON ARZNEIMITTELN MIT HILFE DICHTER GASE**
PROPELLANT MIXTURES AND AEROSOLS FOR MICRONISING MEDICAMENTS WITH COMPRESSED GAS
MELANGES D'AGENTS PROPULSEURS ET AEROSOLS POUR LA MICRONISATION DE MEDICAMENTS A L'AIDE DE GAZ COMPRIMES

(30) Priorität: 25.04.1996 DE 19616573
(43) Veröffentlichungstag der Anmeldung: 21.04.1999
(73) Patentinhaber: PARI GmbH Spezialisten für effektive Inhalation, 82319 Starnberg (DE)
(72) Erfinder: ZHANG, Zhengfeng, D-82327 Tutzing (DE); KNOCH, Martin, D-82335 Berg (DE)
(74) Vertreter: HOFFMANN - EITLE
(86) Internationale Anmeldenummer: PCT/EP1997/002149
(87) Internationale Veröffentlichungsnummer: WO 1997/040824

(56) Entgegenhaltungen:
- EP-A- 0 677 332
- DE-A- 2 741 882
- DE-A- 3 134 723
- US-A- 4 466 838
- US-A- 5 182 097
- PATENT ABSTRACTS OF JAPAN vol. 095, no. 011, 26.Dezember 1995 & JP 07 207257 A (OSAKA ZOSENJO:KK), 8.August 1995,
- PATENT ABSTRACTS OF JAPAN vol. 011, no. 214 (C-434), 10.Juli 1987 & JP 62 033115 A (KAO CORP), 13.Februar 1987,

## Beschreibung

Die Erfindung betrifft Treibmittelmischungen und ihre Verwendung in Arzneimittelaerosolen für die pulmonale Anwendung.

Fluorchlorkohlenwasserstoffe (FCKWs) werden aufgrund ihrer technischen Vorzüge zur Zeit immer noch als Kühlmittel in Kühl- und Klimaanlagen und als Treibgase in Dosieraerosolen eingesetzt.

DE-A-31 34 723 beschreibt Treibgasmittelzusammensetzungen, die 1,1,2-Trichlor-1,2,2-trifluorethan (FCKW 113) Kohlendioxid sowie eine Verbindung aus der Gruppe umfassend Dimethylether, Propan, Isobutan, N-butan und 1,1-Dichlor-1,1,2,2-tetrafluorethan enthalten. Diese Treibmittelmischung wird in Aerosolsprayzusammensetzungen eingesetzt; nach dem Versprühen werden nur Partikelgrößen von 100 µm und mehr erreicht, die nicht lungengängig sind und daher eine Anwendung des Aerosols im pulmonalen Bereich ausschließen. Auch die das im DE-A-27 41 882 offenbarte Aerosoltreibmittelsystem ist für eine Anwendung im pulmonalen Bereich nicht geeignet und wird bevor-' zugt als Treibmittelsystem in einem Antitranspirationsmittel eingesetzt. Es enthält neben 14 bis 32 Gew.% eines Kohlenwasserstofftreibmittels und 0,5 bis 5,3 Gew.% Kohlendioxid als Hauptbestandteil (65 bis 85 Gew.% chlorierte Kohlenwasserstoffe ausgewählt aus 1,1,1-Trichloroethan, Dichloromethan oder Gemischen davon).

Die umweltschädigenden Wirkungen von Fluorchlorkohlenwasserstoffen und Chlorkohlenwasserstoffen, insbesondere hinsichtlich einer Zerstörung der Ozonschicht in der Erdatmosphäre, haben ferner umfangreiche Forschungen nach unbedenklichen Ersatzstoffen ausgelöst. So besteht auch auf dem Gebiet der Aerosolerzeugung das Bedürfnis, neuartige und umweltverträgliche Treibmittel und Herstellungsverfahren für Aeorosole zu entwickeln.

Ein Verfahren zur FCKW-freien Aerosolzubereitung für die Verwendung in speziellen Inhalatoren, sogenannten MDIs ("metered dose inhalers"), wird in US 5190029 offenbart. Als Treibgas dient 1,1,1,2-Tetrafluorethan, auch als R 134a bezeichnet, das entweder allein oder gemischt mit verschiedenen anderen Kohlenwasserstoffen verwendet wird. Der pharmazeutische Wirkstoff ist Salbutamol; als oberflächenaktives Additiv wird Ölsäure zugesetzt. Es wurden die Dichte, Dampfdrücke und andere relevante Eigenschaften der Zubereitungen gemessen, wonach nur 22 % bis 39 % der erzeugten Aerosol-Partikel einen aerodynamischen Durchmesser von kleiner oder gleich 11,2 µm haben.

In WO 9304671 wird ein Aerosol aus einem Arzneistoff, einem Glycerinphosphatid und einem Treibgas oder Treibgasgemisch aus n-Butan, Dimethylether und Propan beschrieben. Die Anwesenheit von Glycerinphosphatiden vergrößert die Löslichkeit des Arzneistoffes im Treibgas. Durch geeignete Einstellung der Verhältnisse der verschiedenen Komponenten bildet das System eine homogene Lösung, also keine Suspension. Ob ausreichend feine Partikel aus dieser homogenen Lösung erzeugt werden können, wird in dieser Anmeldung nicht angegeben.

In mehreren Patentanmeldungen und -schriften werden 1,1,1,2-Tetrafluorethan (R 134a, CH₂FCF₃) und 1,1,1,2,3,3,3-Heptafluorpropan (R 227, CF₃CHFCF₃) als alternative Treibgase vorgeschlagen. Hierzu gehören z.B. DE 4123663, DE 4038203, EP 526002, EP 512502, EP 518601, EP 518600, EP-A-372777, US 5182097, US 5185094, US 5118494, US 5126123, US 5190029, US 5202110, WO 9104011, WO 9200062, WO 9211190 und WO 9206675. Obwohl diese Verbindungen kein Chlor enthalten und deshalb keine schädlichen Auswirkungen auf die Ozonschicht der Erdatmosphäre haben, besitzen sie ein erhebliches Treibhaus-Potential (Michael E. Whitham et al.: Respiratory Drug Delivery IV, 1994, 203; Pamela S. Zurer: Chem. En. News, 15 (11), 1993, 12).

Die handelsüblichen Dosieraerosole mit FCKW liegen in Form einer Suspension des Wirkstoffes im Treibmittel vor. Mit Hilfe einer oder mehrerer oberflächenaktiven Substanzen wird ein Arzneimittel im Treibgasgemisch, das normalerweise aus Dichlordifluormethan (R12), Trichlorfluormethan (R11) und 1,2-Dichlortetrafluorethan (R114) besteht, suspendiert. Die am häufigsten benutzten, oberflächenaktiven Substanzen sind z.B. Sorbitan-Trioleat, Ölsäure und Lecithin. Diese Substanzen zeigen jedoch den Nachteil, daß sie in R134a und R 227 unlöslich sind (Peter R. Byron, et al.: Respiratory Drug Delivery IV, 1994, 237; Ashley Woodcock: Journal of Aerosol Medicine, 8 (suppl. 2) 1995, S. 5).

Verdichtete Gase werden in der Verfahrenstechnik zur Extraktion und Raffination von Naturprodukten bereits seit längerer Zeit eingesetzt. Die Eigenschaften der an diesen Prozessen beteiligten Komponenten und Phasen unter den nahkritischen Bedingungen wurden erst in letzter Zeit stärker erforscht. Die gewonnenen Erkenntnisse eröffneten die Möglichkeiten, dichte Gase auch in anderen Verfahren zu verwenden. Dazu gehört auch der Einsatz in der Medizintechnik zur pulmonalen Arzneimittelapplikation.

In US 5301664 wird ein Verfahren beschrieben, mit dem diverse Wirkstoffe mit Hilfe von verdichtetem Kohlendioxid als feiner Nebel erzeugt werden können. Diese Stoffe werden zunächst in dem überkritischen Kohlendioxid, das einphasig vorliegt, unter einem Druck von 200 bar bei einer Temperatur nahe der Körpertemperatur analog einem Extraktionsprozeß aufgelöst. Durch plötzliche Entspannung beim Austreten aus einer Düse in die Umgebung werden feine Arzneimittelpartikel durch Kondensation infolge der herabgesetzten Lösungsfähigkeit des Lösemittels ausgebildet. Mit der Verwendung eines überkritischen Treibmittelmediums sind jedoch auch erhebliche Nachteile, insbesondere ein hoher apparativer Aufwand verbunden. Das geringe Lösevermögen von Kohlendioxid und die trotz eines eventuellen Schleppmitteleinsatzes erforderlichen sehr hohen Drücke führen zu großen Abmessungen eines solchen Verneblers. Um eine Kondensation in der Druckkammer zu verhindern, die sich zwangsläufig bei der Entleerung einstellen würde, muß der Druck aufrechterhalten werden. Dies geschieht durch einen beweglichen Druckkammerboden, dessen der Druckkammer abgewandte Seite mit einem anderen Druckgas in Verbindung steht. Dieses Druckgas besitzt einen ausreichend hohen Dampfdruck. Zum Einsatz kommt z.B. Stickstoff. Das Druckgas muß dabei den Sicherheitsanforderungen entsprechend in Gasflaschen gelagert werden, die mitgeführt werden müssen. Von einem Handgerät kann daher keine Rede sein. Die Wirkstoffentnahme wird durch ein handbetätigtes Ventil reguliert. Die entnommene Arzneimitteldosis wird daher durch die Öffnungsdauer bestimmt. Auch die Vorlage der Komponenten, d.h. das Befüllen des Verneblers, ist technisch aufwendig.

Die pharmazeutischen Aerosolzubereitungen des Standes der Technik sind somit, insbesondere im Hinblick auf das Treibmittel, verbesserungsbedürftig. Die Aufgabe der vorliegenden Erfindung besteht daher darin, verbesserte und umweltfreundliche Treibmittel und sie enthaltende Aerosolzubereitungen zur Verfügung zu stellen. Erfindungsgemäß soll die Mikronisierung von Arzneimitteln für die pulmonale Verabreichung mit Hilfe dichter Gase, aber unter Vermeidung halogenierter Kohlenwasserstoffe verwirklicht werden. Die so erzeugten Arzneimittelpartikel sollen leicht lungengängig sein, d.h. eine möglichst geringe Partikelgröße aufweisen. Ferner soll der apparative Aufwand und die Handhabung relativ einfach sein, wozu die Arzneimittelmikronisierung bei gemäßigten Bedingungen, d.h. niedrigem Druck und Temperatur (Zimmertemperatur) und mit üblichen Düsenformen realisiert werden soll.

Es ist bekannt, daß einige Arzneimittel in flüssigem Dimethylether, Propan, Butan und anderen Kohlenwasserstoffen löslich sind. Die Dampfdrücke dieser Treibgase bei Zimmertemperatur liegen im Bereich bis ca. 10 bar. Diese Eigenschaft macht sie für eine Aerosolzubereitung geeignet, jedoch besteht ein entscheidendes Problem in ihrer sehr großen Verdampfungsenthalpie. Wenn eine derartige Zubereitung versprüht wird, verdampft das Treibgas nicht ausreichend schnell. Das Arzneimittel kann deshalb nicht sehr fein mikronisiert werden, was die Lungengängigkeit des Arzneimittels behindert.

Die Verdampfungsenthalpie von einigen anderen Gasen, wie z.B. Kohlendioxid, Schwefelhexafluorid und Ethan ist wesentlich kleiner. Aber sie haben im allgemeinen eine sehr schlechte Lösungsfähigkeit und einen relativ hohen Dampfdruck. Wie vorher schon erwähnt, ist z.B. ein Druck von 200 bar oder darüber erforderlich, wenn ein Arzneimittel in einer merklichen Menge in überkritischem Kohlendioxid gelöst wird. Aus diesem Grund sind Kohlendioxid und Schwefelhexafluorid als Treibgase für eine vernünftige und wirtschaftlich günstige Aerosolzubereitung ebenfalls ungeeignet.

Überraschend wurde nunmehr gefunden, daß in einem Arzneimittelaerosol Treibgase mit geringer Verdampfungsenthalpie, wie z.B. Kohlendioxid, Schwefelhexafluorid und Ethan im unterkritischen Zustand verwendet werden können, ohne daß die erwähnten Nachteile auftreten, wenn sie mit einem anderen Gas mit hoher Verdampfungsenthalpie und niedrigem Dampfdruck, wie z.B. Butan, Propan oder Dimethylether, gemischt werden. Alle diese Komponenten sind in flüssigem Zustand miteinander vollständig mischbar. Unerwartet bleiben die günstigen Eigenschaften beider Komponenten erhalten, während sich die ungünstigen Eigenschaften kompensieren. Das zugesetzte Gas hat zwei Funktionen: den Dampfdruck des gesamten Systems zu verringern und die Lösungsfähigkeit des Systems zu vergrößern. Die Verdampfungsenthalpie des Systems ist noch klein genug, so daß keine Probleme bei der Verdampfung auftreten. Außerdem wird die Brennbarkeit des Systems wegen der Anwesenheit von unbrennbarem Gas wesentlich verringert.

Gemäß der Erfindung wird die obige Aufgabe durch eine besondere Treibmittelmischung für Arzneimittelaerosole zur Mikronisierung der Arzneimittel für die pulmonale Anwendung gemäß Anspruch 1 gelöst. Diese Treibmittelmischung liegt im unterkritischen Zustand vor und enthält mindestens eine Komponente aus einer ersten Klasse von Treibgasen und mindestens eine Komponente aus einer zweiten Klasse von Treibgasen, wobei die erste Klasse Treibgase mit einer Verdampfungsenthalpie bei 25 °C von 200 kJ/kg oder weniger und einem Dampfdruck bei 25 °C von über 20 bar, und die zweite Klasse Treibgase mit einer Verdampfungsenthalpie bei 25 °C von 300 kJ/kg oder mehr und einem Dampfdruck bei 25 °C von 10 bar oder weniger umfaßt, und wobei für den Fall, daß eine Komponente aus der ersten Klasse von Treibgasen Kohlendioxid ist, die Treibmittelmischung kein 1,1,2-Trichlor-1,2,2-trifluorethan, 1,1,1-Trichlorethan, Dichlormethan oder Mischungen davon enthält.

Erfindungsgemäß verwendete Vertreter der beiden Treibgasklassen zeichnen sich durch die folgenden Eigenschaften aus:

### Treibgasklasse 1:

Diese Gase besitzen bei Raumtemperatur (25 °C) einen hohen Dampfdruck von 20 bar oder mehr, bevorzugt von 20 bis 70 bar, und eine kleine Verdampfungsenthalpie von 200 kJ/kg oder weniger, bevorzugt 180 bis 50 kJ/kg. Dazu gehören Schwefelhexafluorid, Kohlendioxid und Ethan. Typisch ist ihre schlechte Lösungsfähigkeit für die meisten organischen Substanzen, insbesondere für die Arzneimittel.

### Treibgasklasse 2:

Diese Gase besitzen dagegen einen relativ niedrigen Dampfdruck bei Raumtemperatur (25 °C) von 10 bar oder weniger, bevorzugt 2 bis 10 bar, und eine hohe Verdampfungsenthalpie bei 25 °C von 300 kJ/kg oder mehr, bevorzugt 340 bis 450 kJ/kg. Bevorzugte Vertreter sind Dimethylether, Propan, Butan und Pentan. Häufig zeigen diese Gase eine gute Lösungsfähigkeit für viele organische Substanzen.

Die Erfindung betrifft ferner ein Arzneimittelaerosol für die pulmonale Anwendung, das neben einem oder mehreren Arzneimitteln die oben genannte Treibmittelmischung enthält, und wobei für den Fall, daß eine Komponente aus der ersten Klasse von Treibgasen Kohlendioxid ist, das Arzneimittelaerosol kein 1,1,2-Trichlor-1,2,2-trifluorethan, 1,1,1-Trichlorethan, Dichlormethan oder Mischungen davon enthält.

Die erfindungsgemäßen Treibmittelmischungen enthalten bevorzugt ein oder mehrere Treibgase aus der Treibgasklasse 1 mit einem Anteil, bezogen auf die Treibmittelmischung, von 10 bis 80 Gew.%, besonders bevorzugt 15 bis 60 Gew.% und einem oder mehreren Treibgasen aus der Treibgasklasse 2 mit einem Anteil im Bereich von 20 bis 90 Gew.%, besonders bevorzugt 40 bis 85 Gew.%. Die Treibmittelmischung kann auch noch andere Treibgase enthalten, bevorzugt besteht sie jedoch ausschließlich aus solchen der Klassen 1 und 2. Das Arzneimittel kann in der Aerosolzusammensetzung gelöst (Lösungsaerosol), aber auch suspendiert vorliegen (Suspensionsaerosol).

Für ein Suspensionsaerosol ist auch die Ausgangspartikelgröße des in der Treibmittelmischung zu suspendierenden Arzneimittels von Bedeutung. Da sich bei dem Versprühprozeß die Partikelgröße zwar (unerwünscht) vergrößern, aber praktisch nicht verkleinern kann, sollte das Arzneimittel bereits vor dem Einbringen in das Treibmittel ausreichend fein, d.h. auf einen Partikeldurchmesser von weniger als 8 µm zerkleinert vorliegen. Die feinen Partikel lassen sich auch leichter suspendieren.

Für ein Suspensionsaerosol sind die Treibmittelkombinationen von Schwefelhexafluorid und Butan, von Schwefelhexafluorid und Dimethylether, von Ethan und Butan und von Ethan und Dimethylether wegen ihrer physikalischen Eigenschaften, wie z. B. des Dampfdrucks und der Verdampfungsenthalpie, besonders geeignet. Für ein Lösungsaerosol sind die Treibmittelkombinationen von Schwefelhexafluorid und Dimethylether und von Ethan und Dimethylether wegen der ausgezeichneten Lösungsfähigkeit von Dimethylether besonders geeignet.

Manche Arzneimittel sind in niederen Alkoholen, wie Ethanol, Propanol oder Isopropanol, sowie Wasser, Aceton und einigen anderen Lösemitteln leicht löslich. Zur Verbesserung der Löslichkeit von Arzneimitteln in einem erfindungsgemäß verwendeten Treibgasgemisch in einem Lösungsaerosol können dem solche Verbindungen als Lösungsvermittler oder Schleppmittel zugesetzt werden.

In einem Suspensionsaerosol werden zur verbesserten Suspendierung des Arzneimittels häufig oberflächenaktive Substanzen zugesetzt. Eine Suspensionsaerosolzubereitung setzt voraus, daß die verwendete, oberflächenaktive Substanz in der Treibmittelmischung löslich ist. Die herkömmlichen, oberflächenaktiven Substanzen, wie z.B. Ölsäure, Lecithin und Sorbitan-Trioleat, sind in der Treibgasklasse 2 leicht löslich und auch im hier verwendeten Treibgasgemisch löslich. Deshalb können solche oberflächenaktiven Substanzen ohne Schwierigkeiten bei der Herstellung einer Suspensionsaerosolzubereitung eingesetzt werden.

Die erfindungsgemäße Treibmittelmischung wird im unterkritischen Zustand in einem Arzneimittelaerosol für die pulmonale Anwendung zur Mikronisierung des Arzneimittels verwendet, wobei ca. 80 Gew.-%, bevorzugt ca. 90 Gew.-% und besonders bevorzugt ca. 100 Gew.-% der mikronisierten, d.h. durch Versprühen erzeugten Arzneimittelpartikel einen Durchmesser von weniger als 8 µm haben. In einer weiteren bevorzugten Ausführungsform weisen ca. 80 Gew.-%, bevorzugt ca. 90 Gew.-% und besonders bevorzugt ca. 100 Gew.-% der mikronisierten Arzneimittelpartikel einen Durchmesser von weniger als 5 µm auf. Die Prozentangaben beziehen sich jeweils auf die Gesamtmasse der erzeugten und nach Verdampfen des Treibmittels "getrockneten" Arzneimittelpartikel. Diese Partikel besitzen daher eine kleinere Masse und setzen sich im Mundrohr des Dosieraerosols und im Spacer nicht so leicht ab. Ferner werden durch die verbesserte Lungengängigkeit nicht nur der Bronchial- bzw. obere Lungenbereich, sondern auch tiefer gelegene Teile der Lunge und Lungenbläschen erreicht. Dies ist nicht nur ein entscheidender Vorteil, wenn die Lunge selbst das erkrankte und zu behandelnde Organ darstellt, es wird auch die Resorption von systemisch wirkenden Arzneimitteln verbessert.

Erfindungsgemäß wird die Treibmittelmischung in einer pharmazeutischen Zusammensetzung, nämlich einem Arzneimittelaerosol, zur pulmonalen Anwendung eingesetzt. Der Gehalt der Treibmittelmischung im fertigen Arzneimittelaerosol beträgt bevorzugt 80 bis 99,99 Gew.%, besonders bevorzugt 90 bis 99,99 Gew.%. Diese Zusammensetzung enthält neben dem Arzneimittel, die oben beschriebene Treibmittelmischung und gegebenenfalls weitere übliche pharmazeutisch verträgliche Verdünnungsmittel, Excipienten, Schleppmittel, Lösungsvermittler und oberflächenaktive Mittel. Das Arzneimittel kann in der Aerosolzusammensetzung gelöst oder suspendiert in einem Anteil von 0,01 bis 5 Gew.-%, vorzugsweise von 0,03 bis 1 Gew.-% vorliegen. Der Betriebsdruck der Zusammensetzung beträgt 2 bis 100, vorzugsweise 3 bis 50 bar, besonders bevorzugt 5 bis 20 bar. Zum Mikronisieren wird eine für diesen Zweck übliche Sprühdüse benutzt.

In einer bevorzugten Ausführungsform des neu entwickelten Arzneimittelaerosols besteht die Treibmittelmischung ausschließlich aus einer oder mehreren Komponenten der genannten beiden Klassen. Wahlweise können noch zusätzlich die genannten Lösungsvermittler und/oder oberflächenaktive Substanzen enthalten sein.

Zur Erzielung bestimmter oder verbesserter Auswirkungen kann auch eine Kombination verschiedener Wirkstoffe in unterschiedlichen Anteilen in einer Aerosolzubereitung verwendet werden, wie z. B. Kombinationen aus Ipratropiumbromid und Fenoterol, aus Salbutamol und Dinatriumcromoglicinsäure und aus Salbutamol und Beclometason-17,21-dipropionat.

Bei den Fällen, wo eine oberflächenaktive Substanz verwendet wird, liegt das Gewichtsverhältnis vom Arzneimittel zur oberflächenaktiven Substanz im Bereich von 100/1 bis 1/100, vorzugsweise von 20/1 bis 1/10.

Gemäß der Erfindung haben die pharmazeutischen Aerosolzubereitungen, jeweils bezogen auf die gesamte Zubereitung, sofern nicht anders angegeben, bevorzugt folgende Zusammensetzungen:

| | |
|---|---|
| Treibgasklasse 1 | 10 bis 80 Gew.%, besonders bevorzugt 15 bis 60 Gew.% |
| Treibgasklasse 2 | 20 bis 90 Gew.%, besonders bevorzugt 40 bis 85 Gew.% |
| (jeweils bezogen auf die Treibmittelmischung) | |
| Treibmittelmischung | 80 bis 99,99 Gew.%, besonders bevorzugt 90 bis 99,99 Gew.% |
| Arzneimittel | 0,01 bis 5 Gew.%, besonders bevorzugt 0,03 bis 1 Gew.% |
| oberflächenaktive Substanzen | bis 5 Gew.%, besonders bevorzugt bis 1 Gew.% |
| Lösungsvermittler (Schleppmittel) | bis 10 Gew.%, besonders bevorzugt bis 2 Gew.%. |
| (jeweils bezogen auf fertige Arzneimittelaerosol) | |

Die folgenden Versuchsbeispiele dienen der näheren Erläuterung der Erfindung.

### Anlage und Durchführung der Versuche

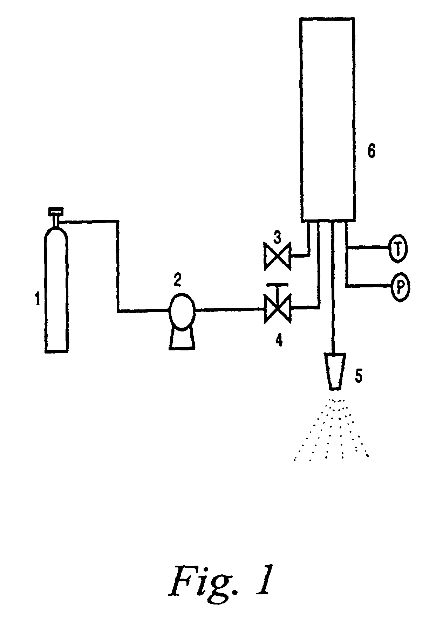

Die Anlage (Abb. 1) besteht aus einer Gasflasche **1** , einer Hochdruckpumpe **2** , einem Sicherheitsventil **3** , einem Rückschlagventil **4** , einer Sprühdüse **5** und einem Autoklaven **6** . **T** ist ein Thermometer und **P** ist ein Manometer. Der Autoklav hat einen Inhalt von 200 ml. Bei einigen Versuchen wird auch ein kleiner Autoklav mit einem Inhalt von 32 ml verwendet. Damit wird eine kleinere Menge von Arzneimitteln und anderen Substanzen benötigt.

Der Autoklav ist in einem Schwingrahmen montiert, welcher an einem Gestell mittels Lagerböcken befestigt ist. Über einen Motor mit Exzenter- und Pleuelstange wird der Rahmen in eine ca. 30° starke Schwingung nach oben und unten versetzt. Nach Reinigung des Autoklaven wird dieser mit einem Medikament und ggf. noch mit Zusatzstoffen gefüllt und mit einem Deckel verschlossen. Dimethylether, Propan oder Butan werden aus der Flasche, die auf einer Waage steht, mit Hilfe der Hochdruckpumpe in den Autoklaven gepumpt. Die Füllmenge kann an der Waage abgelesen werden. Da der Dampfdruck von CO₂, SF₆ bzw. Ethan höher als der Versuchsdruck ist, können diese Stoffe einfach aus einer Flasche in den Autoklaven gefüllt werden.

Nachdem der Druck im Autoklaven den gewünschten Wert erreicht hat, wird dieser ca. 90 Minuten geschüttelt. Anschließend wird er 30 Minuten abgesetzt, so daß sich alle ungelösten Stoffe vom Gasgemisch trennen. Zur Beurteilung der Stabilität einer Suspension wird eine Hochdrucksichtzelle mit einem Volumen von 35 ml verwendet. Die Sichtzelle hat einen großen Sichtdurchmesser von 30 mm, so daß die Beobachtung der Suspension wesentlich erleichtert wird.

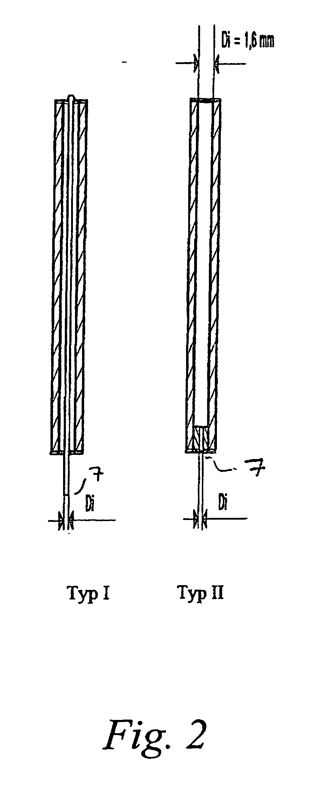

Abb. 2 zeigt verschiedene konventionelle Düsen, die zur Aerosolerzeugung verwendet wurden. Sie unterscheiden sich in Form und Größe der Blendenöffnung 7 (Di ist der jeweilige Innendurchmesser). Zum Typ I gehören die Düsen mit einer Blendenöffnung von 0,35 mm, 0,50 mm, 1,00 mm und 1,60 mm; zum Typ II gehören die Düsen mit einer Blendenöffnung von 0,20 mm, 0,30 mm und 0,50 mm. Der Autoklav wird durch ein Ventil mit einer Düse verbunden. Die Entspannung des verflüssigten Gasgemisches erfolgt über einen Ausgangsdruck im Bereich von 10 bis 40 bar bis zum Umgebungsdruck. Dadurch können kleine Partikel erzeugt werden. Ein Objektträger liegt unter der Düse in einem Abstand von ca. 40 - 100 mm, senkrecht zu der Sprayrichtung. Die Partikel auf dem Objektträger werden mit Hilfe eines Mikroskops (Vergrößerungsfaktor 1000) beobachtet und qualitativ beurteilt.

Die Löslichkeit eines Arzneimittels wird durch Probennahmen ermittelt: Der im Gasgemisch gelöste Feststoff wird nach der Entspannung in ein Reagenzglas abgeschieden, die Gasmenge mit einem Gasvolumenzähler bestimmt. Die abgeschiedene Menge an Feststoff wird gravimetrisch bestimmt, das Verhältnis der beiden Gase gaschromatographisch analysiert.

### Ergebnis und Diskussion

Nach diesem Verfahren werden verschiedene Medikamente erfolgreich zerstäubt. Die Durchmesser aller mikronisierten Arzneimittelpartikel betragen weniger als 8 µm, in einigen Versuchen unter 5 µm bzw. unter 2 µm. Die konkreten Versuchsbedingungen und ihre Ergebnisse werden im folgenden Abschnitt "Beispiel" dargestellt.

Für die Aerosolzubereitung mit einem darin gelösten Arzneimittel ist die in den Autoklaven gefüllte Menge des Wirkstoffes in den meisten Fällen größer als die für eine gesättigte Lösung notwendige Menge. Dies führt zu einer zusätzlichen Feststoffphase, die in diesen Fällen mit der Sichtzelle gesehen werden kann.

Da die Konzentration von Arzneimitteln in Gasgemischen normalerweise wesentlich weniger als 1 Gew.% bezogen auf die Gesamtzusammensetzung ist, hat das Arzneimittel praktisch keinen Einfluß auf das Phasenverhalten des binären Systems. Aufgrund der Tatsache, daß die Phasengleichgewichte in einigen Systemen, wie SF₆/Propan, Dimethylether/CO₂, bekannt sind, kann das Verhältnis von beiden Komponenten in Abhängigkeit von Druck und Temperatur bestimmt werden. Zur Kontrolle werden auch einige Gasproben mit Hilfe der Gaschromatographie analysiert.

Die Löslichkeiten der in dieser Arbeit untersuchten Medikamente in flüssigem Dimethylether betragen meist mehr als 0,1 Gew.%, nur Terbutalinsulfat und Ipratropiumbromid sind etwas schwerer löslich. Die Löslichkeiten in flüssigem n-Butan sind im allgemeinen kleiner als in flüssigem Dimethylether. So betragen die gesättigten Konzentrationen von Budesonid und Beclometason-17,21-dipropionat in n-Butan jeweils ca. 0,06 Gew.% bzw. 0,03 Gew.%.

Durch Zugabe eines Gases aus der Treibgasklasse 1 wird die Löslichkeit verkleinert doch für viele Medikamente sind die Konzentrationen noch so groß, daß sie für die Inhalationstherapie ausreichend sind. So beträgt die Löslichkeit von Salbutamol in flüssigem Dimethylether ca. 0,23 Gew.%. Durch Zugabe von Kohlendioxid nimmt die Löslichkeit ab: Beträgt das Verhältnis CO₂/Dimethylether 39:61, ist der Salbutamol-Gehalt in gesättigtem Zustand des flüssigen Gemisches ca. 0,18 Gew.%. Bei einem Verhältnis von 64:36 beträgt der Salbutamol-Gehalt des flüssigen Gemisches 0,06 Gew.%.

Phospholipide können in einer Aerosolzubereitung als eine oberflächenaktive Substanz fungieren. Sie sind mit flüssigem Dimethylether vollständig mischbar, in flüssigem Propan und Butan leicht löslich, in CO₂, SF₆ und Ethan aber unlöslich.

Bei allen Untersuchungen zur Phasengleichgewichtsmessung und bei den Sprühversuchen entstehen im Autoklaven zwei Fluidphasen, eine Gasphase und eine Flüssigphase, die sich im Gleichgewichtszustand befinden. Sowohl bei der Probenentnahme als auch beim Sprühgang verkleinert sich der Inhalt der Flüssigphase. Solange die Flüssigphase noch vorhanden ist, ändert sich der Druck nicht wesentlich. Bei den untersuchten Bedingungen ist die Dichte der Gasphase gegenüber der Dichte der Flüssigphase sehr klein. Deshalb ändert sich die Zusammensetzung der Flüssigphase sowie ihr Lösungsvermögen während der Aerosolerzeugung nicht merklich. Weil die Konzentration eines Arzneimittels in der Flüssigphase während des Sprühvorgangs stabil ist, wird eine dosisgenaue Aerosolzubereitung ermöglicht. Für die praktische Anwendung ist dies eine entscheidende, günstige Eigenschaft des Systems.

Wie bei den handelsüblichen Dosieraerosolen mit FCKW können verschiedene Schleppmittel oder oberflächenaktive Substanzen, z.B. Ethanol, Wasser, Aceton, Ölsäure und Lipide auch in diesem Verfahren verwendet werden, um die Löslichkeit eines Arzneimittels in dem Gasgemisch zu erhöhen bzw. die Suspension zu stabilisieren.

In Tab. 1 werden die physikalischen Eigenschaften einiger der wichtigsten Treibgase dargestellt. Die erfindungswesentlichen Parameter sind der Dampfdruck und die Verdampfungsenthalpie. Zum Vergleich sind R 11, R 12 und R 114 als die drei zur Zeit in Dosieraerosolen am häufigsten benutzten Treibgase mitaufgenommen.

Erfindungsgemäß wird kein überkritisches Gasgemisch verwendet. Daher kann wegen des relativ niedrigen Dampfdrucks des Gasgemisches die Dimensionierung eines Aerosolbehälters entsprechend klein ausgelegt werden. Die Handhabung wird dadurch vereinfacht.

Die Löslichkeit eines Arzneimittels in einem Treibgasgemisch ist auch von der Temperatur abhängig. Die Temperatur eines Dosieraerosols ist nach oben begrenzt, da das Arzneimittel durch zu hohe Temperatur zerstört werden kann. Für die praktische Anwendung wird daher Raumtemperatur bevorzugt.

Diese Untersuchungen zeigen, daß bei einer Kombination zweier Gase aus den beiden Klassen wesentlich niedrigere Drücke benötigt werden als bei der Verwendung einer überkritischen Komponente. Diese Kombination wirkt sich auch positiv auf die Löslichkeit des Gasgemisches aus. Der Dampfdruck, die Verdampfungsenthalpie und das Lösungsvermögen des Gasgemisches sind in einem großen Bereich dadurch einstellbar, daß das Verhältnis der Komponente mit niedrigem Dampfdruck zu der mit hohem Dampfdruck variiert wird.

Der Betriebsdruck eines Systems ist von dessen Zusammensetzung (Arzneimittel, Gasgemisch und andere Schleppmittel bzw. oberflächenaktive Substanz) abhängig, liegt jedoch im Bereich von 2 bis 100 bar, vorzugsweise 3 bis 50 bar. Für das System Dimethylether/CO₂ liegt der Dampfdruck im Bereich von 5 bis 65 bar, vorzugsweise 30 bis 40 bar. Höherer Druck und kleinere Verdampfungsenthalpie begünstigen die Bildung feiner Partikel beim Sprühprozeß. Für einen höheren Druck muß der CO₂-Gehalt erhöht werden, was zu einem schlechten Lösevermögen des Gemisches führt. Die optimalen Bedingungen sind durch Vorversuche leicht zu ermitteln. Für die praktische Handhabung wird ein niedriger Druck bevorzugt, wofür geeignete Gase verwendet werden können. Durch den Einsatz von Ethan anstelle von CO₂ kann der Betriebsdruck um ca. 20 bar vermindert werden; bei Verwendung von Schwefelhexafluorid sinkt der Druck weiter (Tab. 1).

Die geeignete Blendenöffnung einer Sprühdüse ist von dem Betriebsdruck abhängig. Eine Blendenöffnung von ca. 0,3 bis ca. 0,5 mm, wie sie üblicherweise im für die pulmonale Anwendung eingesetzten Druckbereich verwendet wird, hat sich als günstig erwiesen.

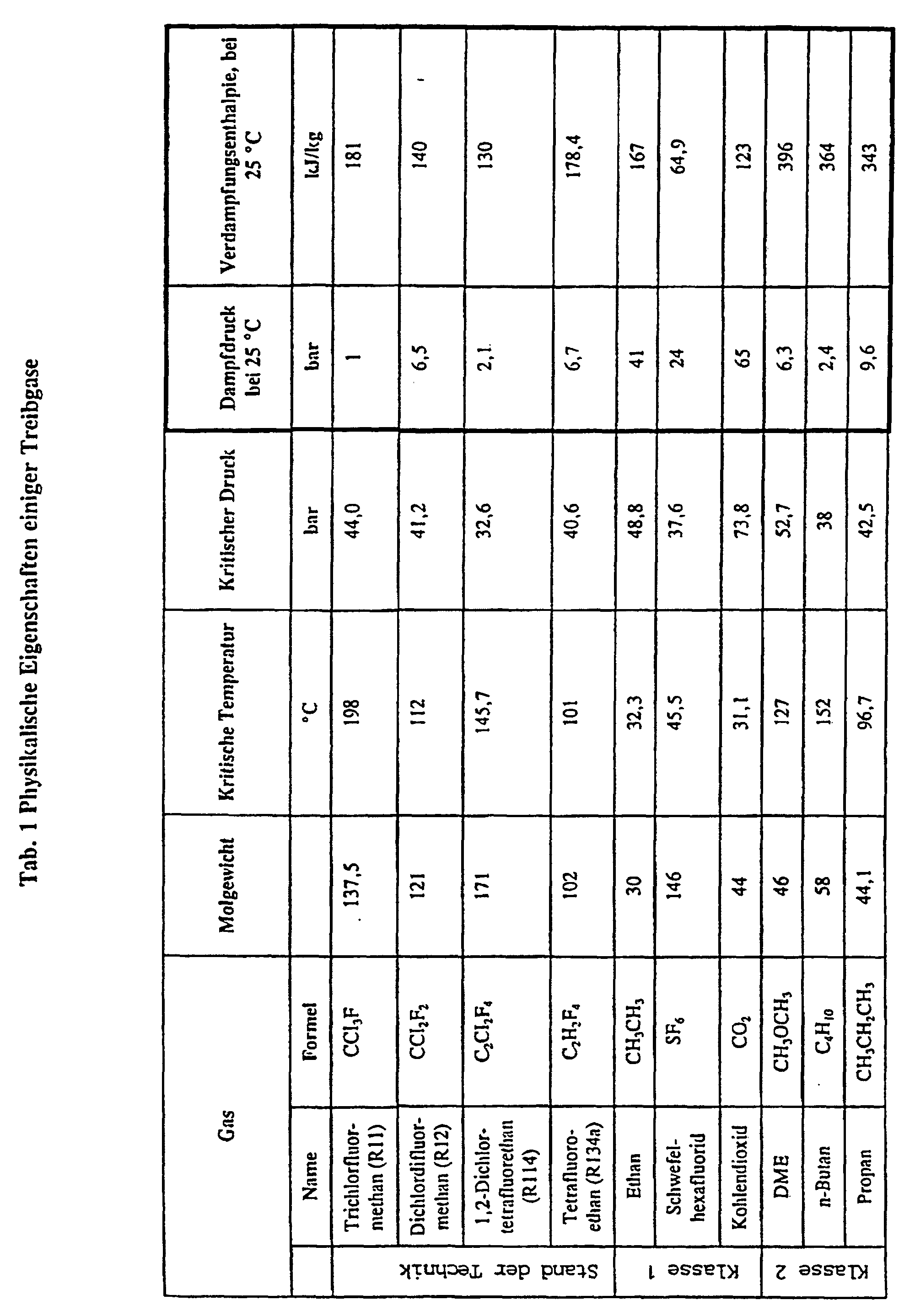

### Beispiel 1

40 mg Budesonid (Budes) und 4 mg Lecithin (Leci) werden in einen Druckbehälter gefüllt. Der Druckbehälter hat einen Inhalt von 32 ml und wird mit einem Manometer und einer Sprühdüse mit einem Durchmesser von 0,50 mm versehen. Durch Evakuieren wird die Luft aus dem Behälter entfernt. Anschließend werden ca. 14 g n-Butan und 6 g SF₆ (Schwe) zugepumpt. Bei 25 °C beträgt der Dampfdruck des Gemisches 10 bar. Durch Schütteln wird Lecithin in diesem flüssigen Gemisch gelöst und Budesonid darin suspendiert. Die gebildete Suspension ist sehr stabil. Erst nach mehreren Minuten wird die Absetzung von Budesonid deutlich beobachtet. Das abgesetzte Budesonid ist durch Schütteln wieder leicht zu suspendieren. Die Suspension wird über ein Entspannungsventil mit Hilfe der Sprühdüse bei einer Entfernung von ca. 5 cm von dem Düsenmund, senkrecht zur Sprayrichtung, auf einen sauberen und trockenen Objektträger gesprüht. Die Partikel auf dem Objektträger werden mit Hilfe eines Mikroskops (Vergrößerungsfaktor 1000) betrachtet. Die überwiegende Mehrheit der Partikel hat einen kleineren Durchmesser als 2 µm. Es gibt sehr wenige Partikel mit einem Durchmesser zwischen 2 und 4 µm. Noch größere Partikel werden nicht gefunden.

### Beispiel 2

24 mg Salbutamol (Salb) und 4 mg Lecithin werden in einen Druckbehälter gefüllt. Der Druckbehälter hat einen Inhalt von 32 ml und wird mit einem Manometer und einer Sprühdüse mit einem Durchmesser von 0,50 mm versehen. Durch Evakuieren wird die Luft im Behälter entfernt. Anschließend werden ca. 8,4 g n-Butan und 3,6 g Ethan zugepumpt. Bei 25 °C beträgt der Dampfdruck des Gemisches 15 bar. Durch Schütteln wird Lecithin in diesem flüssigen Gemisch gelöst und Salbutamol darin suspendiert. Die gebildete Suspension ist sehr stabil. Erst nach mehreren Minuten wird-die Absetzung von Salbutamol deutlich beobachtet. Das abgesetzte Salbutamol ist durch Schütteln wieder leicht zu suspendieren. Die Suspension wird über ein Entspannungsventil mit Hilfe der Sprühdüse bei einer Entfernung von ca. 6 cm von dem Düsenmund, senkrecht zur Sprayrichtung, auf einen sauberen und trockenen Objektträger gesprüht. Die Partikel auf dem Objektträger werden mit Hilfe eines Mikroskops (Vergrößerungsfaktor 1000) betrachtet. Die überwiegende Mehrheit der Partikel hat einen kleineren Durchmesser als 2 µm. Es gibt sehr wenige Partikel mit einem Durchmesser zwischen 2 und 5 µm. Partikel über 5 µm werden nicht gefunden.

### Beispiel 3

180 mg Beclometason-17,21-dipropionat (BDP) werden in einen Autoklaven, wie in Abb. 1 dargestellt wird, gefüllt. Der Autoklav hat einen Inhalt von 200 ml und wird mit einem Manometer und einer Sprühdüse mit einem Durchmesser von 0,30 mm versehen. Nach der Evakuierung werden ca. 78 g Dimethylether (DME) und 78 g SF₆ zugepumpt. Bei 25 °C beträgt der Dampfdruck des Gemisches 20 bar. Durch Schütteln wird Beclometason-17,21-dipropionat in diesem flüssigen Gemisch gelöst. Die gebildete homogene Lösung wird über ein Entspannungsventil mit Hilfe der Sprühdüse bei einer Entfernung von ca. 6 cm von dem Düsenmund, senkrecht zur Sprayrichtung, auf einen sauberen und trockenen Objektträger gesprüht. Die Partikel auf dem Objektträger werden mit Hilfe eines Mikroskops (Vergrößerungsfaktor 1000) betrachtet. Die überwiegende Mehrheit der Partikel hat einen kleineren Durchmesser als 5 µm. Es gibt sehr wenige Partikel mit einem Durchmesser über 5 µm.

### Beispiel 4

50 mg Terbutalinsulfat (Terbu) und 5 mg Lecithin werden in einen Druckbehälter gefüllt. Der Druckbehälter hat einen Inhalt von 32 ml und wird mit einem Manometer und einer Sprühdüse mit einem Durchmesser von 0,50 mm versehen. Durch Evakuieren wird die Luft im Behälter entfernt. Anschließend werden ca. 8 g n-Butan und 3 g Ethan zugepumpt. Bei 25 °C beträgt der Dampfdruck des Gemisches 15 bar. Durch Schütteln wird Lecithin in diesem flüssigen Gemisch gelöst und Terbutalinsulfat darin suspendiert. Die gebildete Suspension ist stabil. Erst nach mehreren Minuten wird die Absetzung von Terbutalinsulfat deutlich beobachtet. Das abgesetzte Terbutalinsulfat ist durch Schütteln wieder leicht zu suspendieren. Die Suspension wird über ein Entspannungsventil mit Hilfe der Sprühdüse bei einer Entfernung von ca. 6 cm von dem Düsenmund, senkrecht zur Sprayrichtung, auf einen sauberen und trockenen Objektträger gesprüht. Die Partikel auf dem Objektträger werden mit Hilfe eines Mikroskops (Vergrößerungsfaktor 1000) betrachtet. Die überwiegende Mehrheit der Partikel hat einen kleineren Durchmesser als 3 µm. Es gibt sehr wenige Partikel mit einem Durchmesser zwischen. 3 und 6 µm. Partikel mit einem Durchmesser über 6 µm werden nicht gefunden.

### Beispiel 5

0,1 g Dinatriumcromoglicinsäure (DNCG) wird in einen Autoklaven, wie in Abb. 1 dargestellt wird, gefüllt. Der Autoklav hat einen Inhalt von 200 ml und wird mit einem Manometer und einer Sprühdüse mit einem Durchmesser von. 0,35 mm versehen. Nach der Evakuierung werden ca. 20 g Ethan und 70 g DME zugepumpt. Bei 25 °C beträgt der Dampfdruck des Gemisches 18 bar. Durch Schütteln wird Dinatriumcromoglicinsäure in diesem flüssigen Gemisch gelöst.

Die gebildete homogene Lösung wird über ein Entspannungsventil mit Hilfe der Sprühdüse bei einer Entfernung von ca. 6 cm von dem Düsenmund, senkrecht zur Sprayrichtung, auf einen sauberen und trockenen Objektträger gesprüht. Die Partikel auf dem Objektträger werden mit Hilfe eines Mikroskops (Vergrößerungsfaktor 1000) betrachtet. Die überwiegende Mehrheit der Partikel hat einen kleineren Durchmesser als 6 µm. Es gibt sehr wenige Partikel mit einem Durchmesser über 6 µm.

### Beispiel 6

10 mg Ipratropiumbromid (Iprat) und 2 mg Lecithin werden in einen Druckbehälter gefüllt. Der Druckbehälter hat einen Inhalt von 32 ml und wird mit einem Manometer und einer Sprühdüse mit einem Durchmesser von 0,50 mm versehen. Durch Evakuieren wird die Luft aus dem Behälter entfernt. Anschließend werden ca. 13 g n-Butan und 5 g SF₆ zugepumpt. Bei 25 °C beträgt der Dampfdruck des Gemisches 9 bar. Durch Schütteln wird Lecithin in diesem flüssigen Gemisch gelöst und Ipratropiumbromid darin suspendiert. Die gebildete Suspension ist stabil. Erst nach mehreren Minuten wird die Absetzung von Ipratropiumbromid deutlich beobachtet. Das abgesetzte Ipratropiumbromid ist durch Schütteln wieder leicht zu suspendieren. Die Suspension wird über ein Entspannungsventil mit Hilfe der Sprühdüse bei einer Entfernung von ca. 5 cm von dem Düsenmund, senkrecht zur Sprayrichtung, auf einen sauberen und trockenen Objektträger gesprüht. Die Partikel auf dem Objektträger werden mit Hilfe eines Mikroskops (Vergrößerungsfaktor 1000) betrachtet. Die überwiegende Mehrheit der Partikel hat einen kleineren Durchmesser als 4 µm. Es gibt sehr wenige Partikel mit einem Durchmesser über 4 µm.

### Beispiel 7

25 mg Salbutamol werden in einen Druckbehälter gefüllt. Der Druckbehälter hat einen Inhalt von 32 ml und wird mit einem Manometer und einer Sprühdüse mit einem Durchmesser von 0,50 mm versehen. Durch Evakuieren wird die Luft aus dem Behälter entfernt. Anschließend werden ca. 12 g DME und 8 g SF₆ zugepumpt. Bei 25 °C beträgt der Dampfdruck des Gemisches 17 bar. Durch Schütteln wird Salbutamol in diesem flüssigen Gemisch gelöst. Die gebildete homogene Lösung wird über ein Entspannungsventil mit Hilfe der Sprühdüse bei einer Entfernung von ca. 5 cm von dem Düsenmund, senkrecht zur Sprayrichtung, auf einen sauberen und trockenen Objektträger gesprüht. Die Partikel auf dem Objektträger werden mit Hilfe eines Mikroskops (Vergrößerungsfaktor 1000) betrachtet. Die überwiegende Mehrheit der Partikel hat einen kleineren Durchmesser als 5 µm. Es gibt sehr wenige Partikel mit einem Durchmesser über 5 µm.

### Beispiel 8

60 mg Amphotericin B (Amph) und 2 mg Lecithin werden in einen Druckbehälter gefüllt. Der Druckbehälter hat einen Inhalt von 32 ml und wird mit einem Manometer und einer Sprühdüse mit einem Durchmesser von 0,50 mm versehen. Durch Evakuieren wird die Luft aus dem Behälter entfernt. Anschließend werden ca. 8 g Dimethylether und 12 g SF₆ zugepumpt. Bei 25 °C beträgt der Dampfdruck des Gemisches 21 bar. Durch Schütteln wird Lecithin in diesem flüssigen Gemisch gelöst und Amphotericin B darin suspendiert. Die gebildete Suspension ist sehr stabil. Erst nach mehreren Minuten wird die Absetzung von Amphotericin B deutlich beobachtet. Das abgesetzte Amphotericin B ist durch Schütteln wieder leicht zu suspendieren. Die Suspension wird über ein Entspannungsventil mit Hilfe der Sprühdüse bei einer Entfernung von ca. 5 cm von dem Düsenmund, senkrecht zur

Sprayrichtung, auf einen sauberen und trockenen Objektträger gesprüht. Die Partikel auf dem Objektträger werden mit Hilfe eines Mikroskops (Vergrößerungsfaktor 1000) betrachtet. Die überwiegende Mehrheit der Partikel hat einen kleineren Durchmesser als 2 µm. Es gibt sehr wenige Partikel mit einem Durchmesser zwischen 2 und 6 µm. Partikel mit einem Durchmesser über 6 µm werden nicht gefunden.

### Beispiel 9

15 mg Salbutamol, 10 mg Beclometason-17,21-dipropionat und 4 mg Lecithin werden in einen Druckbehälter gefüllt. Der Druckbehälter hat einen Inhalt von 32 ml und wird mit einem Manometer und einer Sprühdüse mit einem Durchmesser von 0,50 mm versehen. Durch Evakuieren wird die Luft aus dem Behälter entfernt. Anschließend werden ca. 8,5 g n-Butan und 3,8 g Ethan zugepumpt. Bei 25 °C beträgt der Dampfdruck des Gemisches 15 bar. Durch Schütteln wird Lecithin in diesem flüssigen Gemisch gelöst und Salbutamol und Beclometason-17,21-dipropionat darin suspendiert. Die gebildete Suspension ist sehr stabil. Erst nach mehreren Minuten wird die Absetzung von den Arzneimitteln deutlich beobachtet. Die abgesetzten Arzneimittel sind durch Schütteln wieder leicht zu suspendieren. Die Suspension wird über ein Entspannungsventil mit Hilfe der Sprühdüse bei einer Entfernung von ca. 6 cm von dem Düsenmund, senkrecht zur Sprayrichtung, auf einen sauberen und trockenen Objektträger gesprüht. Die Partikel auf dem Objektträger werden mit Hilfe eines Mikroskops (Vergrößerungsfaktor 1000) betrachtet. Die überwiegende Mehrheit der Partikel hat einen kleineren Durchmesser als 3 µm. Es gibt sehr wenige Partikel mit einem Durchmesser zwischen 3 und 5 µm. Partikel mit einem Durchmesser über 5 µm werden nicht gefunden.

### Vergleichsbeispiel 1

40 mg Budesonid werden in einen Druckbehälter gefüllt. Der Druckbehälter hat einen Inhalt von 32 ml und wird mit einem Manometer und einer Sprühdüse mit einem Durchmesser von 0,50 mm versehen. Durch Evakuieren wird die Luft aus dem Behälter entfernt. Anschließend werden ca. 16 g Dimethylether zugepumpt. Bei 25 °C beträgt der Dampfdruck des Systems 6 bar. Durch Schütteln wird Budesonid in flüssigem Dimethaylether gelöst. Die homogene Lösung wird über ein Entspannungsventil mit Hilfe der Sprühdüse bei einer Entfernung von ca. 5 cm von dem Düsenmund, senkrecht zur Sprayrichtung, auf einen sauberen und trockenen Objektträger gesprüht. Die Partikel auf dem Objektträger werden mit Hilfe eines Mikroskops (Vergrößerungsfaktor 1000) betrachtet. Die Durchmesser der dadurch erzeugten Budesonidpartikel sind sehr unterschiedlich: einige Partikel haben einen kleineren Durchmesser als 10 µm, die meisten Partikel haben einen Durchmesser im Bereich 10 bis 30 µm, einige Partikel über 30 µm.

### Vergleichsbeispiel 2

60 mg Amphotericin B und 4 mg Lecithin werden in einen Druckbehälter gefüllt. Der Druckbehälter hat einen Inhalt von 32 ml und wird mit einem Manometer und einer Sprühdüse mit einem Durchmesser von 0,50 mm versehen. Durch Evakuieren wird die Luft aus dem Behälter entfernt. Anschließend werden ca. 17 g Dimethylether zugepumpt. Bei 25 °C beträgt der Dampfdruck des Systems 6 bar. Durch Schütteln wird Lecithin in diesem flüssigen Gemisch gelöst, und Amphotericin B darin suspendiert. Die gebildete Suspension ist sehr stabil. Erst nach mehreren Minuten wird die Absetzung von Amphotericin B deutlich beobachtet. Das abgesetzte Amphotericin B ist durch Schütteln auch wieder leicht zu suspendieren. Die Suspension wird über ein Entspannungsventil mit Hilfe der Sprühdüse bei einer Entfernung von ca. 5 cm von dem Düsenmund , senkrecht zur Sprayrichtung, auf einen sauberen und trockenen Objektträger gesprüht. Die Partikel auf dem Objektträger werden mit Hilfe eines Mikroskops (Vergrößerungsfaktor 1000) betrachtet. Die Durchmesser der dadurch erzeugten Partikel sind sehr unterschiedlich: einige Partikel haben einen kleineren Durchmesser als 6 µm, die meisten Partikel haben einen Durchmesser im Bereich 6 bis 20 µm, einige Partikel über 20 µm, teilweise sogar über 50 µm.

### Vergleichsbeispiel 3

40 mg Salbutamol und 8 mg Lecithin werden in einen Druckbehälter gefüllt. Der Druckbehälter hat einen Inhalt von 32 ml und wird mit einem Manometer und einer Sprühdüse mit einem Durchmesser von 0,50 mm versehen. Durch Evakuieren wird die Luft im Behälter entfernt. Anschließend werden ca. 30 g Schwefelhexafluorid zugepumpt. Bei 25 °C beträgt der Dampfdruck des Systems 24 bar. Durch Schütteln werden weder Lecithin noch Salbutamol in flüssigem Schwefelhexafluorid gelöst oder suspendiert. Sie flottieren schnell auf die Oberfläche wegen deren leichteren spezifischen Gewichts im Vergleich zu dem von Schwefelhexafluorid. Das Gemisch wird über ein Entspannungsventil mit Hilfe der Sprühdüse bei einer Entfernung von ca. 6 cm von dem Düsenmund, senkrecht zur Sprayrichtung, auf einen sauberen und trockenen Objektträger gesprüht. Wenn die Zeit zwischen dem Schütteln der Zubereitung und dem Versprühen über 10 Sekunden beträgt, werden praktisch kein Salbutamol und Lecithin versprüht. Wenn das Versprühen nach dem Schütteln sofort stattfindet, werden sehr wenige feine Partikel mit einem Durchmesser im Bereich von ca. 1 bis 3 µm auf dem Objektträger gefunden.

Die Ergebnisse der Beispiele 1 bis 9 und der Vergleichsbeispiele 1 bis 3 sind in der Tabelle 2 zusammengefaßt. Hierbei ergibt sich, daß erst bei einer Kombination von Treibgasen aus den beiden genannten Klassen die gewünschten feinen Partikelgrößen erhalten werden. Wird nur ein Treibgas aus einer Klasse verwendet, werden gar keine oder ungenügend Partikel erhalten, oder die Partikelgröße ist inakzeptabel hoch.

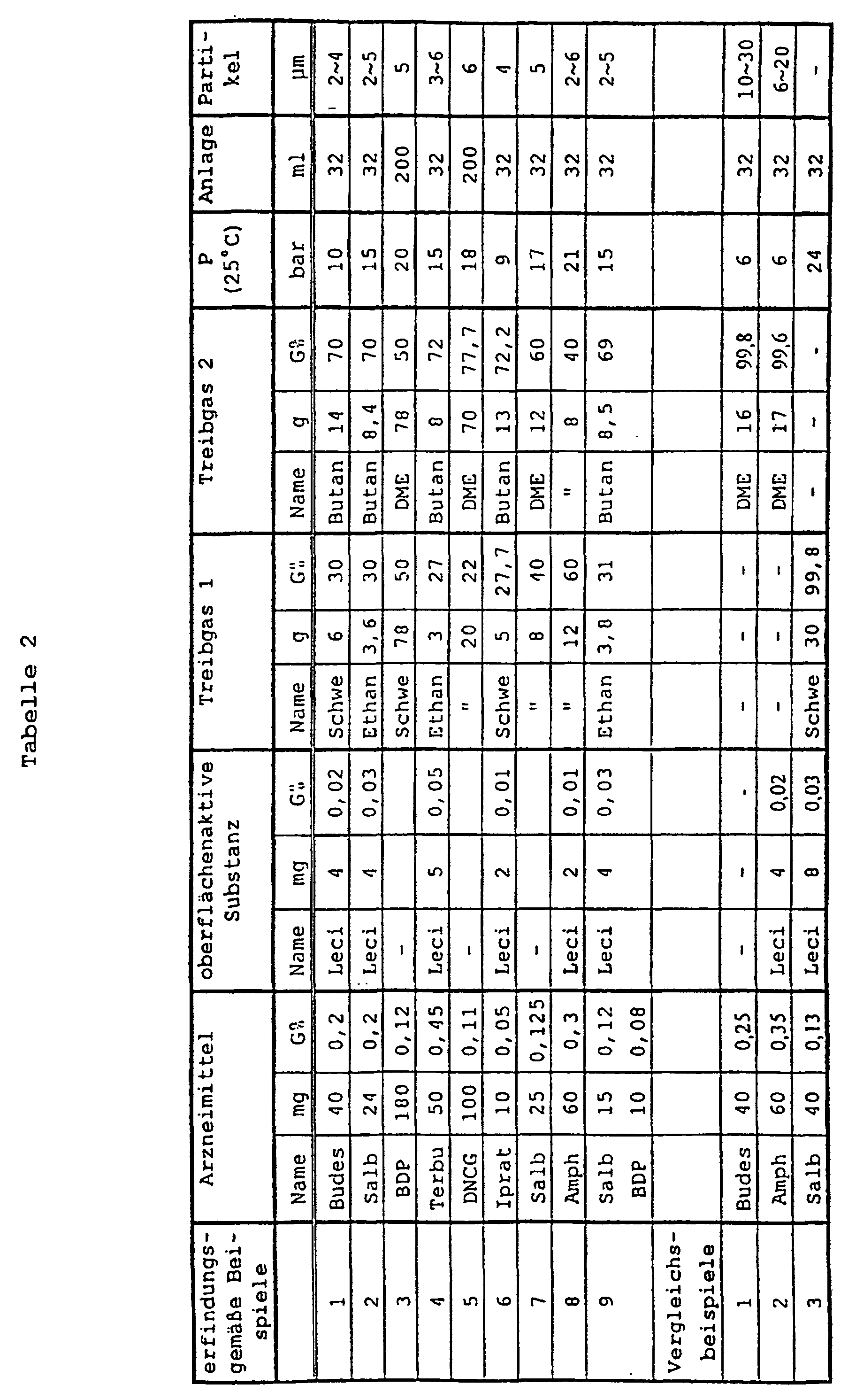

## Patentansprüche

1. Treibmittelmischung für Arzneimittelaerosole zur Mikronisierung der Arzneimittel für die pulmonale Anwendung, **dadurch gekennzeichnet, daß** die Treibmittelmischung im unterkritischen Zustand vorliegt, keine halogenierten Kohlenwasserstoffe enthält, und mindestens eine Komponente aus einer ersten Klasse von Treibgasen und mindestens eine Komponente aus einer zweiten Klasse von Treibgasen enthält, wobei die erste Klasse Treibgase mit einer Verdampfungsenthalpie bei 25°C von 200 kJ/kg oder weniger und einem Dampfdruck bei 25°C von 20 bar oder mehr, und die zweite Klasse Treibgase mit einer Verdampfungsenthalpie bei 25°C von 300 kJ/kg oder mehr und einem Dampfdruck bei 25°C von 10 bar oder weniger umfasst, worin der Anteil der ersten Klasse von Treibgasen 10 bis 80 Gew.%, bezogen auf die Treibmittelmischung, beträgt und worin der Anteil der zweiten Klasse von Treibgasen 20 bis 90 Gew.%, bezogen auf die Treibmittelmischung, beträgt.

2. Treibmittelmischung nach Anspruch 1, worin die erste Klasse Treibgase mit einer Verdampfungsenthalpie bei 25 °C von 50 bis 180 kJ/kg und einem Dampfdruck bei 25 °C von 20 bis 70 bar umfaßt.

3. Treibmittelmischung nach Anspruch 1, worin die zweite Klasse Treibgase mit einer Verdampfungsenthalpie bei 25 °C von 340 bis 450 kJ/kg und einem Dampfdruck bei 25 °C von 2 bis 10 bar umfaßt.

4. Treibmittelmischung nach Anspruch 1, worin die Treibgase der ersten Klasse aus Kohlendioxid, Schwefelhexafluorid und Ethan ausgewählt sind.

5. Treibmittelmischung nach Anspruch 1, worin die Treibgase der zweiten Klasse aus Dimethylether, Propan, Butan und Pentan ausgewählt sind.

6. Treibmittelmischung nach einem der Ansprüche 1 bis 5, worin die Treibmittelmischung aus einem oder mehreren Treibgasen der ersten Klasse und einem oder mehreren Treibgasen der zweiten Klasse besteht.

7. Treibmittelmischung nach einem der Ansprüche 1 bis 6, worin die Treibmittelmischung für ein Suspensionsaerosol Treibmittelkombinationen ausgewählt aus Schwefelhexafluorid und Butan, aus Schwefelhexafluorid und Dimethylether, aus Ethan und Butan, und aus Ethan und Dimethylether umfaßt.

8. Treibmittelmischung nach einem der Ansprüche 1 bis 6, worin die Treibmittelmischung für ein Lösungsaerosol die Treibmittelkombination ausgewählt aus Schwefelhexafluorid und Dimethylether, und aus Ethan und Dimethylether umfaßt.

9. Arzneimittelaerosol zur Mikronisierung des Arzneimittels für die pulmonale Anwendung **dadurch gekennzeichnet, daß** das Arzneimittelaerosol frei von halogenierten Kohlenwasserstoffen ist und eine Treibmittelmischung nach einem oder mehreren der Ansprüche 1 bis 8 enthält.

10. Arzneimittelaerosol nach Anspruch 9, worin das Arzneimittel in dem Arzneimittelaerosol gelöst oder suspendiert in einem Anteil von 0,01 bis 5 Gew.-%, bezogen auf das Arzneimittelaerosol, vorliegt.

11. Arzneimittelaerosol nach Anspruch 9 oder 10, worin die Treibmittelmischung in einem Anteil von 80 bis 99,99 Gew.%, bezogen auf das Arzneimittelaerosol, vorliegt.

12. Arzneimittelaerosol nach einem der Ansprüche 9 bis 11, worin das Arzneimittel ausgewählt ist aus den Wirkstoffen Budesonid, Salbutamol, Beclometason-17,21-dipropionat, Terbutalinsulfat, Dinatriumcromoglicinsäure, Ipratropiumbromid, Amphotericin B und Kombinationen davon.

13. Arzneimittelaerosol nach einem der Ansprüche 9 bis 12, worin das Arzneimittelaerosol zusätzlich Lösungsvermittler für das Arzneimittel enthält.

14. Arzneimittelaerosol nach Anspruch 13, worin die Lösungsvermittler aus Alkoholen, Wasser und Aceton ausgewählt sind.

15. Arzneimittelaerosol nach einem der Ansprüche 9 bis 14, worin das Arzneimittelaerosol zusätzlich oberflächenaktive Substanzen enthält.

16. Arzneimittelaerosol nach Anspruch 15, worin die oberflächenaktiven Substanzen aus Ölsäure, Lecithin und Sorbitan-Trioleat ausgewählt sind.

17. Arzneimittelaerosol nach einem der Ansprüche 9 bis 16, worin der Betriebsdruck des Arzneimittelaerosols 2 bis 100 bar beträgt.

18. Arzneimittelaerosol nach Anspruch 17, worin der Betriebsdruck des Arzneimittelaerosols 3 bis 50 bar beträgt.

19. Verwendung einer unterkritischen Treibmittelmischung nach einem der Ansprüche 1 bis 8 in einem Arzneimittelaerosol, das keine halogenierten Kohlenwasserstoffe enthält, für die pulmonale Anwendung zur Mikronisierung des Arzneimittels, wobei ca. 80 Gew.-% bevorzugt ca. 90 Gew.-%, besonders bevorzugt ca. 100 Gew.-% der mikronisierten Arzneimittelpartikel einen Durchmesser von weniger als 8 µm haben.

20. Verwendung nach Anspruch 19, worin ca. 80 Gew.-%, bevorzugt ca. 90 Gew.-%, besonders bevorzugt ca. 100 Gew.-% der mikronisierten Arzneimittelpartikel einen Durchmesser von weniger als 5 µm haben.

21. Verwendung nach Anspruch 19 oder 20, worin die Treibmittelmischung in einem Anteil von 80 bis 99,99 Gew.-%, bezogen auf das Arzneimittelaerosol, vorliegt.

22. Verwendung nach einem der Ansprüche 19 bis 21, worin das Arzneimittel in dem Arzneimittelaerosol gelöst oder suspendiert in einem Anteil von 0,01 bis 5 Gew.-%, bezogen auf das Arzneimittelaerosol, vorliegt.

23. Verwendung nach einem der Ansprüche 19 bis 22, worin das Arzneimittel ausgewählt ist aus den Wirkstoffen Budesonid, Salbutamol, Beclometason -17,21-dipropionat,Terbutalinsulfat, Dinatriumcromoglicinsäure, Ipratropiumbromid, Amphotericin B und Kombinationen davon.

24. Verwendung nach einem der Ansprüche 19 bis 23, worin das Arzneimittelaerosol zusätzlich Lösungsvermittler für das Arzneimittel enthält.

## Claims

1. Propellant mixture for medicament aerosols for micronisation of the medicaments for pulmonary administration, **characterised in that** the propellant mixture is present in subcritical state, contains no halogenated hydrocarbons, and contains at least one component from a first class of propellant gases and at least one component from a second class of propellant gases, wherein the first class comprises propellant gases having an evaporation enthalpy at 25°C of 200 kJ/kg or less and a vapour pressure at 25°C of 20 bar or more, and the second class comprises propellant gases having an evaporation enthalpy at 25°C of 300 kJ/kg or more and a vapour pressure at 25°C of 10 bar or less, wherein the proportion of the first class of propellant gases is 10 to 80 wt.%, based on the propellant mixture, and wherein the proportion of the second class of propellants gases is 20 to 90 wt.%, based on the propellant mixture.

2. Propellant mixture according to claim 1, wherein the first class comprises propellant gases having an evaporation enthalpy at 25°C of 50 to 180 kJ/kg and a vapour pressure at 25°C of 20 to 70 bar.

3. Propellant mixture according to claim 1, wherein the second class comprises propellant gases having an evaporation enthalpy at 25°C of 340 to 450 kJ/kg and a vapour pressure at 25°C of 2 to 10 bar.

4. Propellant mixture according to claim 1, wherein the propellant gases of the first class are selected from carbon dioxide, sulphur hexafluoride and ethane.

5. Propellant mixture according to claim 1, wherein the propellant gases of the second class are selected from dimethyl ether, propane, butane and pentane.

6. Propellant mixture according to one of claims 1 to 5, wherein the propellant mixture consists of one or more propellant gases of the first class and one or more propellant gases of the second class.

7. Propellant mixture according to one of claims 1 to 6, wherein the propellant mixture for a suspension aerosol comprises propellant combinations selected from sulphur hexafluoride and butane, from sulphur hexafluoride and dimethyl ether, from ethane and butane, and from ethane and dimethyl ether.

8. Propellant mixture according to one of claims 1 to 6, wherein the propellant mixture for a solution aerosol comprises the propellant combination selected from sulphur hexafluoride and dimethyl ether, and from ethane and dimethyl ether.

9. Medicament aerosol for micronisation of the medicament for pulmonary administration, **characterised in that** the medicament aerosol is free of halogenated hydrocarbons and contains a propellant mixture according to one or more of claims 1 to 8.

10. Medicament aerosol according to claim 9, wherein the medicament is present in the medicament aerosol dissolved or suspended in a proportion of 0.01 to 5 wt.%, based on the medicament aerosol.

11. Medicament aerosol according to claim 9 or 10, wherein the propellant mixture is present in a proportion of 80 to 99.99 wt.%, based on the medicament aerosol.

12. Medicament aerosol according to one of claims 9 to 11, wherein the medicament is selected from the active ingredients budesonid, salbutamol, beclomethasone-17,21-dipropionate, terbutalin sulphate, disodium cromoglycinic acid, ipratropium bromide, amphotericin B and combinations thereof.

13. Medicament aerosol according to one of claims 9 to 12, wherein the medicament aerosol additionally contains solubilisers for the medicament.

14. Medicament aerosol according to claim 13, wherein the solubilisers are selected from alcohols, water and acetone.

15. Medicament aerosol according to one of claims 9 to 14, wherein the medicament aerosol additionally contains surfactant substances.

16. Medicament aerosol according to claim 15, wherein the surfactant substances are selected from oleic acid, lecithin and sorbitan trioleate.

17. Medicament aerosol according to one of claims 9 to 16, wherein the operating pressure of the medicament aerosol is 2 to 100 bar.

18. Medicament aerosol according to claim 17, wherein the operating pressure of the medicament aerosol is 3 to 50 bar.

19. Use of a subcritical propellant mixture according to one of claims 1 to 8 in a medicament aerosol, which contains no halogenated hydrocarbons, for pulmonary administration for micronisation of the medicament, wherein about 80 wt.%, preferably about 90 wt.%, particularly preferably about 100 wt.%, of the micronised medicament particles have a diameter of less than 8 µm.

20. Use according to claim 19, wherein about 80 wt.%, preferably about 90 wt.%, particularly preferably about 100 wt.%, of the micronised medicament particles have a diameter of less than 5 µm.

21. Use according to claim 19 or 20, wherein the propellant mixture is present in a proportion of 80 to 99.99 wt.%, based on the medicament aerosol.

22. Use according to one of claims 19 to 21, wherein the medicament is present in the medicament aerosol dissolved or suspended in a proportion of 0.01 to 5 wt.%, based on the medicament aerosol.

23. Use according to one of claims 19 to 22, wherein the medicament is selected from the active ingredients budesonid, salbutamol, beclomethasone-17, 21-dipropionate, terbutalin sulphate, disodium cromoglycinic acid, ipratropium bromide, amphotericin B and combinations thereof.

24. Use according to one of claims 19 to 23, wherein the medicament aerosol additionally contains solubilisers for the medicament.

## Revendications

1. Mélange d'agents propulseurs pour des aérosols médicamentaires, pour la micronisation des médicaments pour l'application pulmonaire, **caractérisé en ce que** le mélange d'agents propulseurs se présente à l'état sous-critique, ne contient aucun hydrocarbure halogéné et contient au moins un composant issu de la première classe des gaz propulseurs, et au moins un composant issu de la deuxième classe des gaz propulseurs, la première classe des gaz propulseurs présente une enthalpie d'évaporation à 25°C de 200 kJ/kg ou moins et une pression de vapeur à 25°C de 20 bar ou plus, et la deuxième classe de gaz propulseurs présente une enthalpie d'évaporation à 25°C de 300 kJ/kg ou plus et une pression de vapeur à 25°C de 10 bar ou moins, dans lequel la proportion de la première classe de gaz propulseurs est de 10 à 80 % en poids, en se référant au mélange d'agents propulseurs et dans lequel la proportion de la deuxième classe de gaz propulseurs est de 20 à 90 % en poids, en se référant au mélange d'agents propulseurs.

2. Mélange d'agents propulseurs selon la revendication 1, la première classe de gaz propulseurs présentant une enthalpie d'évaporation à 25°C de 50 à 180 kJ/kg et une pression de vapeur à 25°C de 20 à 70 bar.

3. Mélange d'agents propulseurs selon la revendication 1, dans lequel la deuxième classe de gaz propulseurs présentant une enthalpie d'évaporation à 25°C de 340 à 450 kJ/kg et une pression de vapeur à 25°C de 2 à 10 bar.

4. Mélange d'agents propulseurs selon la revendication 1, dans lequel les gaz propulseurs de la première classe sont sélectionnés parmi le dioxyde carbone, l'hexafluorure de soufre et l'éthane.

5. Mélange d'agents propulseurs selon la revendication 1, dans lequel les gaz propulseurs de la deuxième classe sont sélectionnés parmi le diméthyl-éther, propane, butane et pentane.

6. Mélange d'agents propulseurs selon l'une des revendications 1 à 5, dans lequel le mélange d'agents propulseurs est composé d'un ou plusieurs gaz propulseurs de la première classe et d'un ou plusieurs gaz propulseurs de la deuxième classe.

7. Mélange d'agents propulseurs selon l'une des revendications 1 à 6, dans lequel le mélange d'agents propulseurs, pour obtenir un aérosol de mise en suspension, contient des combinaisons d'agents propulseurs sélectionnés parmi l'hexafluorure de soufre et le butane, parmi l'hexafluorure de soufre et le diméthyl-éther, parmi l'éthane et le butane et parmi l'éthane et le diméthyl-éther.

8. Mélange d'agents propulseurs selon l'une des revendications 1 à 6, dans lequel le mélange d'agents propulseurs, pour obtenir un aérosol de dissolution, présente la combinaison d'agents propulseurs sélectionné parmi l'hexafluorure de soufre et le diméthyl-éther et parmi l'éthane et le diméthyl-éther.

9. Aérosol médicamentaire pour la micronisation du médicament, pour application pulmonaire, **caractérisé en ce que** l'aérosol médicamentaire est exempt d'hydrocarbures halogénés et contient un mélange d'agents propulseurs selon l'une ou plusieurs des revendications 1 à 8.

10. Aérosol médicamentaire selon la revendication 9, dans lequel le médicament se trouvant dans l'aérosol médicamentaire se présente à l'état dissout ou mis en suspension, en une proportion de 0,01 à 5 % en poids, en se référant à l'aérosol médicamentaire.

11. Aérosol médicamentaire selon la revendication 9 ou 10, dans lequel le mélange d'agents propulseurs se présente en une proportion de 80 à 99,99 % en poids, par rapport à l'aérosol médicamentaire.

12. Aérosol médicamentaire selon l'une des revendications 9 à 11, dans lequel le médicament est sélectionné parmi les principes actifs que sont le budesonide, le salbutamol, le 17,21-dipropionate de béclométasone, le terbutaline sulfate, l'acide disodiumcromoglycinique, le bromure d'ipratropium, l'amphotéricine B et leurs combinaisons.

13. Aérosol médicamentaire selon l'une revendications 9 à 12, dans lequel l'aérosol médicamentaire contient en plus un agent de solubilisation pour le médicament.

14. Aérosol médicamentaire selon la revendication 13, dans lequel les agents de solubilisation sont sélectionnés parmi les alcools, l'eau et l'acétone.

15. Aérosol médicamentaire selon l'une des revendications 9 et 14, dans lequel l'aérosol médicamentaire contient en plus des substances tensio-actives.

16. Aérosol médicamentaire selon la revendication 15, dans lequel les substances tensio-actives sont sélectionnées parmi l'acide oléique, la lécithine et le trioléate de sorbitane.

17. Aérosol médicamentaire selon l'une des revendications 9 à 16, dans lequel la pression de fonctionnement de l'aérosol médicamentaire est de 2 à 100 bar.

18. Aérosol médicamentaire selon la revendication 17, dans lequel la pression de fonctionnement de l'aérosol médicamentaire est de 3 à 50 bar.

19. Utilisation d'un mélange d'agents propulseurs sous-critique, selon l'une des revendications 1 à 8, dans un aérosol médicamentaire, qui ne contient aucun hydrocarbure halogéné, pour l'application pulmonaire, pour la micronisation du médicament, sachant qu'environ 80 % en poids, de préférence environ 90 % en poids, de façon particulièrement préférée environ 100 % en poids des particules de médicaments micronisés, ont un diamètre inférieur à 8 µm.

20. Utilisation selon la revendication 19, dans laquelle environ 80 % en poids, de préférence environ 90 % en poids, de façon particulièrement préférée environ 100 % en poids des particules médicamentaires micronisées, ont un diamètre inférieur à 5 µm.

21. Utilisation selon la revendication 19 ou 20, dans laquelle le mélange d'agents propulseurs se présente en une proportion de 80 à 99,99 % en poids, en se référant à l'aérosol médicamentaire.

22. Utilisation selon l'une des revendications 19 à 21, dans laquelle le médicament se présente dans l'aérosol médicamentaire, à l'état dissout ou mis en suspension, en une proportion de 0,01 à 5 % en poids, en se référant à l'aérosol médicamentaire.

23. Utilisation selon l'une des revendications 19 à 22, dans laquelle le médicament est sélectionné parmi les principes actifs que sont la budesonide, le salbutamol, le 17,21-dipropionate de béclométasone, le sulfate de terbutaline, l'acide disodiumcromoglycinique, le bromure d'ipratropium, l'amphotéricine B et leurs combinaisons.

24. Utilisation selon l'une des revendications 19 à 23, dans laquelle l'aérosol médicamentaire contient en plus des agents de solubilisation pour le médicament.
